# EUROPÄISCHE PATENTSCHRIFT

(11) **EP 1 313 497 B1**
(45) Veröffentlichungstag und Bekanntmachung des Hinweises auf die Patenterteilung: **23.06.2010**
(21) Anmeldenummer: 01969619.4
(22) Anmeldetag: 21.08.2001
(51) Int. Cl.: A61K 36/482, A61K 8/97

(54) **VERWENDUNG VON EXTRAKTEN DER PFLANZE CASSIA ALATA**
USE OF EXTRACTS OF THE CASSIA ALATA PLANT
UTILISATION D'EXTRAITS DE LA PLANTE CASSIA ALATA

(30) Priorität: 29.08.2000 FR 0011040
(43) Veröffentlichungstag der Anmeldung: 28.05.2003
(73) Patentinhaber: Cognis France, S.A.S., 31360 Boussens (FR)
(72) Erfinder: DANOUX, Louis, F-54420 Saulxures les Nancy (FR); PAULY, Gilles, F-54000 Nancy (FR); MOSER, Philippe, F-54270 Essey-Les-Nancy (FR)
(74) Vertreter: Fabry, Bernd
(86) Internationale Anmeldenummer: PCT/EP2001/009602
(87) Internationale Veröffentlichungsnummer: WO 2002/017938

(56) Entgegenhaltungen:
- US-A- 5 466 455
- DATABASE WPI Week 199302 Derwent Publications Ltd., London, GB; AN 1992-012130 XP002172491 & JP 04 338313 A (NONOGAWA SHOJI), 25. November 1992 (1992-11-25)
- DATABASE CHEMABS [Online] CHEMICAL ABSTRACTS SERVICE, COLUMBUS, OHIO, US; Database accession no. 2001:107871 XP002172487 & JP 2001 039823 A (ICHIMARU PHARCOS INC.) 13. Februar 2001 (2001-02-13)
- DATABASE BIOSIS [Online] BIOSCIENCES INFORMATION SERVICE, PHILADELPHIA, PA, US; Database accession no. PREV198069073723 XP002172488 & R.M. SMITH ET AL.: "Anthraquinones from the leaves of cassia alata from fiji" NEW ZEALAND JOURNAL OF SCIENCE, Bd. 22, - 1979 Seiten 123-126,
- DATABASE BIOSIS [Online] BIOSCIENCES INFORMATION SERVICE, PHILADELPHIA, PA, US; Database accession no. PREV198273044797 XP002172489 & T.V. BENJAMIN ET AL.: "Investigation of cassia alata a plant used in nigeria in the treatment of skin diseases" QUARTERLY JOURNAL OF CRUDE DRUG RESEARCH, Bd. 19, - 1996 Seiten 93-96,
- DATABASE BIOSIS [Online] BIOSCIENCES INFORMATION SERVICE, PHILADELPHIA, PA, US; Database accession no. PREV199192079912 XP002172490 & S. PALANICHAMY ET AL.: "wound healing activity of cassia alata" FITOTERAPIA, Bd. 62, - 1991 Seiten 153-156,
- DATABASE WPI Week 199534 Derwent Publications Ltd., London, GB; AN 1995-261166 XP002172492 & JP 07 165527 A (NIPPON SHINYAKU & SHISEIDO), 27. Juni 1995 (1995-06-27)
- DATABASE WPI Week 200138 Derwent Publications Ltd., London, GB; AN 2001-360289 XP002187591 & JP 2001 081021 A (ICHIMARU PHARCOS), 27. März 2001 (2001-03-27)
- DATABASE WPI Week 199812 Derwent Publications Ltd., London, GB; AN 1998-126434 XP002187592 & JP 10 008049 A (SHISEIDO), 13. Januar 1998 (1998-01-13)

## Beschreibung

### Gebiet der Erfindung

Die Erfindung befindet sich auf dem Gebiet der Pflegestoffe und betrifft die Verwendung von Pflanzenextrakten der Pflanze Cassia alata in der Kosmetik.

### Stand der Technik

Kosmetische Zubereitungen stehen dem Verbraucher heute in einer Vielzahl von Kombinationen zur Verfügung. Dabei wird nicht nur erwartet, dass diese Kosmetika einen bestimmten pflegenden Effekt zeigen oder einen bestimmten Mangel beheben, sondern immer häufiger wird nach Produkten verlangt, die mehrere Eigenschaften gleichzeitig aufweisen und somit ein verbessertes Leistungsspektrum zeigen. Ebenso darf der Anwender erwarten, dass die Zusammensetzung des Produktes eine optimale dermatologische Verträglichkeit besitzt, so dass auch empfindliche Verbraucher nicht mit Irritation reagieren. Darüber hinaus sollten die Mittel jedoch auch weitere Funktionen erfüllen, die zunehmend im Bereich der Pflege und insbesondere der Protektion liegen. Von besonderem Interesse sind Stoffe, die sowohl Wirkstoffe darstellen, die für Haut beispielsweise pflegende, vor Alterserscheinungen schützende, revitalisierende Eigenschaften vermitteln als auch gleichzeitig die technischen Eigenschaften des kosmetischen Produktes, wie Lagerstabilität, Lichtstabilität und Formulierbarkeit positiv beeinflussen oder zumindest nicht verschlechtern. Hierbei sind zusätzlich eine gute Hautverträglichkeit und besonders der Einsatz natürlicher Produkte beim Kunden gefragt. Daneben ist es wünschenswert, durch Kombination bereits bekannter Wirkstoffe, oder durch auffinden neuer Einsatzgebiete bereits bekannter Substanzklassen deutlich bessere Produkte zu erhalten. Ein Nachteil besteht hier allerdings häufig darin, das eine Kombination von Wirkstoffen erst dann erhalten wird, wenn unterschiedliche Pflanzenextrakte gleichzeitig in unterschiedlichen Mengenverhältnissen verwendet werden.

Extrakte von Pflanzen und deren Inhaltstoffe finden immer häufiger Einsatz in der Kosmetik und Dermatologie. Pflanzenextrakte werden seit vielen Jahren in den unterschiedlichsten Kulturen für medizinische aber auch bereits für kosmetische Zwecke genutzt. Oftmals waren für diese Pflanzenextrakte nur ganz bestimmte einzelne Wirkungen bekannt und das Einsatzgebiet sehr eingeschränkt.

### Beschreibung der Erfindung

Die Aufgabe der vorliegenden Patentanmeldung hat darin bestanden,

Pflanzenextrakte aus einer Pflanze für die kosmetische Anwendung bereitzustellen, die vorbeugende Wirkung bei Alterserscheinungen der Haut zeigen.

Gegenstand der Erfindung ist die Verwendung von Extrakten der Blätter der Pflanze Cassia gemäß Ansprüchen 1 und 2. Überraschenderweise wurde gefunden, dass durch den Einsatz von Extrakten aus Cassia alata Produkte erhalten werden, die gleichzeitig gute pflegende und schützende Eigenschaften für die Haut, sowie eine hohe Hautverträglichkeit besitzen. Die so erhaltenen Mittel zeichnen sich durch besonders gute Effekte in der Hautkosmetik aus. Sie zeigen neben antioxidativen und anti-inflammatorischen Eigenschaften auch eine vorbeugende und heilende Wirkung bei Alterserscheinungen der Haut und eine revitalisierenden und reaktivierende Aktivität auf die Haut.

Diese mehrfachen Einsatzgebiete der erfindungsgemäßen Mittel aus dem nachwachsendem Rohstoff der Pflanze Cassia alata macht es für den Markt und für den Verbraucher sehr attraktiv.

### Cassia alata

Die erfindungsgemäß einzusetzenden Extrakte werden aus Pflanzen der Gattung Caesalpiniaceae, speziell aus der selteneren Art Cassia alata gewonnen. Die Cassia Arten werden auch unter der Bezeichnung Gewürzrinde zusammengefasst. Häufig sind die Arten Cassia angustifolia, Cassia acutifolia sowie Cassia senna. Bei der Pflanze Cassia alata handelt es sich um einen krautigen dicken Strauch von 2 bis 3 m Höhe, dessen Blätter 30 bis 60 cm lang werden. Die gefiederten Blätter stehen wechselseitig mit 8-14 Paaren und sind länglich stumpf mit einer Länge von 5-15 cm und einer Breite von 3-8 cm. Die Blüten erscheinen an kurzen Blütenstengeln und die Knospen sind eingehüllt in gelben Deckblättern.

Sie ist in den Tropen weit verbreitet und erstreckt sich von tropischen Gebieten Amerikas über Afrika, Indien, Indonesien und Malaysia. Die erfindungsgemäß einzusetzende Pflanze kann aus einem dieser genannten Gebiete stammen.

In der traditionellen indischen Medizin hat diese Pflanze bereits Anwendung gefunden zur Therapie von Husten und Asthma. Auch gegen Schlangenbisse wurde diese Pflanze bereits verabreicht. Als Mittel gegen Schlangenbisse werden die frischen Blätter innerlich angewendet. Die Blätter wurden in Malaysia angewendet als Medizin gegen die Ringwurmerkrankung. In Indonesien werden die zerstampften frischen Blätter von Cassia alata gegen Herpes eingesetzt. In einer Veröffentlichung im Journal of Ethnopharmacology wird über analgetische Eigenschaften des 85%-Ethanolextraktes aus entfetteten Blättern aus Cassia alata berichtet (vergleiche: Palanichamy S., Nagarajan S.; Journal of Ethnopharmacology; 1990, 29, 73-78). Von den gleichen Autoren wird in der Zeitschrift Fitoterapia eine antibakterielle Aktivität des 85%-igen Ethanolextraktes der entfetteten Blätter beschrieben (Palanichamy S., Amala Bhaskar E., Nagarajan S.; Fitoterapia; 1991; 62; 249-252). Eine antifungische Aktivität eines 95%-igen Ethanolextraktes der entfetteten Blätter von Cassia alata gegen Pilze der Gattung Trichophyton und Microsporum wurde von Ibrahim und Osman gefunden und in dem Journal of ethnopharmacology veröffentlicht (Ibrahim D., Osman H.A.; Journal of ethnopharmacology; 1995, 45, 151-156).

Die Extrakte der Pflanze sind bekannt als medizinische Wirkstoffe sowohl in der traditionellen Medizin als auch in der heutigen Forschung. Ihre antimikrobielle und analgetische Wirkung konnte bereits nachgewiesen werden.

### Extraktion

Die Herstellung der erfindungsgemäß einzusetzenden Extrakte erfolgt durch übliche Methoden der Extraktion von Pflanzen bzw. Pflanzenteilen. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei beispielhaft auf Hagers Handbuch der Pharmazeutischen Praxis, (5. Auflage, Bd. 2, S. 1026-1030, Springer Verlag, Berlin-Heidelberg-New-York 1991) verwiesen. Als Ausgangsmaterial können frische oder getrocknete Blätter eingesetzt werden, üblicherweise wird jedoch von Blättern ausgegangen, die vor der Extraktion mechanisch zerkleinert und gegebenenfalls entfettet werden. Hierbei eignen sich alle dem Fachmann bekannten Zerkleinerungsmethoden, als Beispiel sei die Zerkleinerung mit einem, mit Messern versetzten enthaltenen Gerät genannt.

Als Lösungsmittel für die Durchführung der Extraktionen können vorzugsweise organische Lösungsmittel, Wasser oder Gemische aus organischen Lösungsmitteln und Wasser, insbesondere niedermolekulare Alkohole, Ester, Ether, Ketone oder halogenhaltige Kohlenwasserstoffe mit mehr oder weniger hohen Wassergehalten (destilliert oder nicht destilliert) vorzugsweise wässrig, alkoholische Lösungen mit mehr oder weniger hohen Wassergehalten, verwendet werden. Besonders bevorzugt ist die Extraktion mit Wasser, Methanol, Ethanol, Propanol, Butanol und deren Isomere, Aceton, Propylenglycolen, Polyethylenglycolen Ethylacetat, Dichlormethan, Trichlormethan sowie Mischungen hieraus. Die Extraktion erfolgt in der Regel bei 20 bis 100 °C, bevorzugt bei 80 bis 100°C, insbesondere bei 80 bis 90 °C. In einer möglichen Ausführungsform erfolgt die Extraktion unter Inertgasatmosphäre zur Vermeidung der Oxidation der Inhaltsstoffe des Extraktes. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem gewünschten Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Pflanzen oder getrockneter Pflanzenteile gegebenenfalls entfettet, liegen im Bereich von 10 bis 20, vorzugsweise 12 bis 19, insbesondere 13 bis 16 Gew.-%. Die vorliegende Erfindung umfasst die Erkenntnis, dass die Extraktionsbedingungen sowie die Ausbeuten der Endextrakte je nach gewünschtem Einsatzgebiet gewählt werden können. Falls gewünscht, können die Extrakte anschließend beispielsweise einer Sprüh- oder Gefriertrocknung unterworfen werden.

Die Einsatzmenge der Pflanzenextrakte in den genannten Zubereitungen richtet sich nach der Konzentration der einzelnen Inhaltstoffe und nach der Art der Anwendungen der Extrakte. Die Gesamtmenge des Pflanzenextraktes, der in den erfindungsgemäßen Zubereitungen enthalten ist, beträgt in der Regel 0,001 bis 25 Gew.-%, vorzugsweise 0,01 bis 5 Gew.-%, insbesondere 0,05 bis 1,5 Gew.-% bezogen auf die Endzubereitung, mit der Maßgabe, dass sich die Mengenangaben mit Wasser und gegebenenfalls weiteren Hilfs- und Zusatzstoffen zu 100 Gew.-% addieren.

Die erfindungsgemäßen Extrakte enthalten einen Wirkstoffgehalt in den Extrakten von 5 bis 100 Gew.-%, vorzugsweise 10 bis 95 Gew.-%, insbesondere 20 bis 80 Gew.-%. Der Wirkstoffgehalt im Sinne der Erfindung bezeichnet die Summe aller im Extrakt vorhandenen Wirkstoffe bezogen auf das Trockengewicht des Extraktes.

Wirkstoff im Sinne der Erfindung bezieht sich auf die im Extrakt enthaltenen Inhaltstoffe auch wenn deren Gehalt und Identität mit herkömmlichen, dem Fachmann bekannten Methoden noch nicht nachzuweisen sind. Unter Wirkstoffe im Sinne der Erfindung sind weiterhin alle im Extrakt erhaltenen Inhaltsstoffe zu verstehen, deren Wirkung entweder bereits bekannt ist, oder deren Wirkung mit herkömmlichen, dem Fachmann bekannten Methoden noch nicht nachgewiesen werden konnte.

Aktivsubstanz im Sinne der Erfindung bezieht sich auf den Anteil an Substanzen sowie Hilfs- und Zusatzstoffen, die in dem Mittel enthaltend sind, mit Ausnahme des zusätzlich hinzugefügten Wassers.

Der Gesamtanteil an Hilfs- und Zusatzstoffe kann 1 bis 50, vorzugsweise 5 bis 40 Gew.-% - bezogen auf die Endzubereitung der kosmetischen Zubereitungen - betragen. Die Herstellung der Zubereitungen kann durch übliche Kalt - oder Heißprozesse erfolgen; vorzugsweise arbeitet man nach der Phaseninversionstemperatur-Methode.

### Extrakte:

Die erfindungsgemäßen Extrakte der Blätter der Pflanze Cassia alata enthalten in der Regel Substanzen aus der Gruppe bestehend aus Flavonderivaten insbesondere Kämpferol und Kämpferol-Derivaten, Tanninen, Coumarinen, Anthrachinonen, sowie freie Phenolsäure insbesondere p-Hydroxybenzoesäure. Die Extrakte sind je nach gewähltem Ausgangsmaterial und nach gewählter Extraktionsmethode unterschiedlich zusammengesetzt.

Im Sinne der vorliegenden Erfindung sind unter Flavonderivaten solche zu verstehen, die sich aus der Pflanze Cassia alata isolieren lassen. Im Besonderen handelt es sich um Stoffe, die Hydrierungs-, Oxidations- oder Substitutionsprodukte des 2-Phenyl-4H-1-benzopyrans darstellen, wobei eine Hydrierung in der 2,3-Stellung des Kohlenstoffgerüsts bereits vorliegen kann, eine Oxidation in der 4-Stellung bereits vorliegen kann, und unter Substitutionsprodukte der Ersatz eines oder mehrerer Wasserstoffatome durch Hydroxy- oder Methoxy-Gruppen zu verstehen ist. Bei dieser Definition sind also Flavane, Flavan-3-ole (Catechine), Flavan-3,4-diole (Leukoanthocyanidine), Flavone, Flavonole und Flavanone im herkömmlichen Sinn eingeschlossen. Als besonders bevorzugte Flavonderivate isoliert aus der Pflanze Cassia alata gelten Kämpferol und Kämpferolderivate wie zum Beispiel Kämpferol-3-O-sophorosid, Kämpferol-7-rhamnosid, Kämpferol-3-7-difiamnosid.

Unter Tanninen sind im Sinne der vorliegenden Erfindung solche zu verstehen, die sich aus der Pflanze Cassia alata isolieren lassen. Im Besonderen handelt es sich um Polyphenole, die aufgrund ihrer Abstammung von Gallussäure auch als Gallotannine bezeichnet werden. Sie stellen Gemische von Stoffen vom Typ der Pentadigalloylglucose (C76H52O46, MR 1701,22) dar. Es handelt sich weiterhin um Stoffe, die durch oxidative Kupplung der Galloyl-Reste in 1,2,3,4,6-Pentagalloyl-D-glucose entstehen, sowie um deren Folgeprodukte.

Im Sinne der vorliegenden Erfindung sind unter **Cumarinen** solche zu verstehen, die sich aus der Pflanze Cassia alata isolieren lassen. Die Bezeichnung Cumarin gilt als Synonym und ist mit den Bezeichnungen Coumarin, Chromen-2-on, Kumarin, 2 H-1-Benzopyran-2-on, o-Cumar(in)säurelacton und Tonkabohnencampher gleichzusetzen. Cumarin stellt das Cyclisierungsprodukt aus der Cumarinsäure dar. Cumarinssäure ist die ortho-Hydroxyzimtsäure. Im Sinne der Erfindung ist unter Cumarin auch das Glucosid der Cumarinsäure zu verstehen.

Unter Anthrachinonen sind im Sinne der Erfindung solche zu verstehen, die sich aus der Pflanze Cassia alata isolieren lassen. Im Besonderen handelt es sich um Anthrachinon oder um Stoffe, die Oxidations- oder Substitutionsprodukte des 9,10-Anthracendions darstellen, wobei unter Substitutionsprodukte der Ersatz eines oder mehrerer Wasserstoffatome durch Hydroxy- oder MethylGruppen zu verstehen ist. Insbesondere handelt es sich um Alizarin, Chinizarin, Chrysazin, Hystazarin, Purpurin, Chrysophansäure, Chinalizarin und Flavopurpurin.

Im Sinne der Erfindung sind unter freien Phenolsäuren solche zu verstehen, die sich aus der Pflanze Cassia alata isolieren lassen. Bevorzugt darunter zu verstehen sind p-Hydroxybenzoesäure und o-Hydroxybenzoesäure oder Salicylsäure.

### Pflegemittel:

Als Pflegemittel im Sinne der Erfindung sind Pflegemittel für die Haut zu verstehen. Diese Pflegemittel schließen unter anderem stimulierende, heilende und aufbauende Wirkung für die Haut ein. Als Pflegemittel im Sinne der Erfindung sind bevorzugt solche Pflegemittel zu verstehen, die einen stimulierenden Effekt auf die Hautzellen und deren Funktionen besitzen sowie weiterhin aufbauende Wirkung für die Haut und vorbeugende Wirkung gegen Umwelteinflüsse für die Haut zeigen. Weiterhin sind im Sinne der Erfindung bevorzugt Pflegemittel als solche zu verstehen, die verschiedene Erkrankungen der Haut mit ihren unterschiedlichen Auswirkungen auf Aussehen und Funktion der Haut entweder verbessern oder heilen können. Prinzipiell kann man die efindungsgemäßen Extrakte in allen kosmetischen Produkten zur topischen Verwendung einsetzen. Beispiele für kosmetische Produkte sind in ihren Formulierungen in den Tabelle 12 bis 15 beschrieben.

Die vorliegende Erfindung schließt die Erkenntnis ein, dass durch das Zusammenwirken der Inhaltsstoffe der Pflanzenextrakte, insbesondere der oben genannten, besonders wirkungsvolle kosmetische Mittel erhalten werden.

Die efindungsgemäßen Zubereitungen zeigen eine hervorragende hautpflegende Wirkung bei gleichzeitig hoher Hautverträglichkeit. Außerdem zeigen sie eine gute Stabilität, insbesondere gegenüber oxidativer Zersetzung der Produkte.

Die Begriffe Zubereitungen, Endzubereitungen und Mittel sind im Sinne der Erfindung mit dem Begriff Pflegemittel gleichzusetzen.

Aktivsubstanz im Sinne der Erfindung bezieht sich auf den Anteil an Substanzen sowie Hilfs- und Zusatzstoffen, die in den Zubereitungen enthaltend sind, mit Ausnahme des zusätzlich hinzugefügten Wassers.

Ein Gegenstand der Erfindung ist die kosmetische Verwendung von Extrakten aus Cassia alata Blättern in Pflegemitteln zur vorbeugenden Behandlung von Alterserscheinungen der Haut. Eine andere Bezeichnung für diese Art der Pflegemittel ist auch anti-ageing Mittel. Zu diesen Alterserscheinungen zählen beispielsweise jede Art der Fältchen- und Faltenbildung. Die Behandlungen schließen eine Verlangsamung von Altersprozessen der Haut mit ein. Die Alterserscheinungen können die unterschiedlichsten Ursachen aufweisen. Insbesondere sind diese Alterserscheinungen auf Grund einer durch UV-Strahlung induzierten Schädigung der Haut verursacht. In einer besonderen Ausführungsform der Erfindung werden diese Pflegemittel zur Behandlung von durch UV-Strahlung induzierten Alterserscheinungen der Haut eingesetzt. In einer weiteren besonderen Ausführungsform der Erfindung werden diese Pflegemittel zur Behandlung von induzierter Apoptose und damit induzierten Alterserscheinungen der Haut eingesetzt, die durch einen Mangel an Wachstumsfaktoren hervorgerufen wurden.

Im Sinne der Erfindung versteht man unter Apoptose den gezielten Zelltod bestimmter unerwünschter oder geschädigter Zellen. Es handelt sich um einen aktiven Prozess der Zellen (Selbstmord auf Befehl). Apoptose wird durch einen oxidativen Stress (UV-Strahlung, Entzündung), durch einen Mangel an Wachstumsfaktoren oder durch giftige Stoffe (Schmutzstoffe, genotoxische Stoffe usw.) eingeleitet. Bei der Hautalterung z. B. kann es durch einen Mangel an Wachstumsfaktoren in der Haut zu einer induzierten Apoptose der Hautzellen kommen. Bei den durch Apoptose betroffenen Zellen wird durch das spezifische Enzym Endonuclease die nukleare DNA abgebaut und die DNA-Fragmente in das Cytoplasma geschleust. Als Wachstumsfaktoren im Sinne der Erfindung sind prinzipiell alle körpereigenen oder von außen zugeführten zu verstehen, die das Wachstum von Haut- und Haarzellen stimulieren. Dazu zählen beispielsweise Hormone und chemische Mediatoren oder Signalmoleküle. Es handelt sich zum Beispiel um Polypeptid-Wachstumsfaktoren oder Glykoprotein-Wachstumsfaktoren. Hier sei der Epidermale Wachstumsfaktor (EGF) genannt, der aus 53 Aminosäuren besteht und damit einen Polypeptid Wachstumsfaktor darstellt oder das Fibrillin, welches zu den Glykoproteinen gehört. Weitere Wachstumsfaktoren sind beispielsweise Urogastron, Laminin, Follistatin und Heregelin.

### Sonnenschutzmittel bzw. UV-Lichtschutzfaktoren

Als Sonnenschutzmittel bzw. UV-Lichtschutzfaktoren im Sinne der Erfindung werden Lichtschutzmittel bezeichnet, die für den Schutz der menschlichen Haut gegenüber schädigenden Einflüssen der direkten und indirekten Strahlung der Sonne nützlich sind. Die für die Hautbräunung verantwortliche Ultravioleftstrahlung der Sonne unterteilt man in die Abschnitte UV-C (Wellenlängen 200-280 nm), UV-B (280-315 nm) und UV-A (315-400 nm).

Die Pigmentierung normaler Haut unter dem Einfluss der Sonnenstrahlung, d. h. die Bildung von Melaninen, wird durch UV-B und UV-A unterschiedlich bewirkt. Bestrahlung mit UV-A-Strahlen ("langwelligem UV") hat die Dunkelung der in der Epidermis bereits vorhandenen Melanin-Körper zur Folge, ohne dass schädigende Einflüsse zu erkennen sind. Anders bei dem sog. "kurzwelligen UV" (UV-B). Dieses bewirkt die Entstehung von sog. Spätpigment durch Neubildung von Melanin-Körnem. Ehe jedoch das (schützende) Pigment gebildet ist, unterliegt die Haut der Einwirkung der ungefilterten Strahlung, die - je nach Expositionsdauer - zur Bildung von Hautrötungen (Erythemen), Hautentzündungen (Sonnenbrand) und gar Brandblasen führen kann.

Als UV-Absorber oder Lichtfilter, die also die UV-Strahlung in unschädliche Wärme umwandeln, werden Extrakte der Pflanze Cassia alata eingesetzt, diese können zusätzlich in Kombination mit weiteren Sonnenschutzmitteln bzw. UV-Lichtschutzfaktoren vorliegen.

Diese weiteren UV- Lichtschutzfaktoren sind beispielsweise bei Raumtemperatur flüssig oder kristallin vorliegende organische Substanzen (Lichtschutzfilter), die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-isopropylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexylester;
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzophenon-5-sulfonsäure und ihre Salze;
➢ Sulfonsäurederivate des 3-Benzylidencamphers, wie z.B. 4-(2-Oxo-3-bomylidenmethyl)benzolsulfonsäure und 2-Methyl-5-(2-oxo-3-bomyliden)sulfonsäure und deren Salze.

Als typische UVA-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen, wie beispielsweise beschrieben in der DE 19712033 A1 (BASF). Die UV-A und UV-B-Filter können selbstverständlich auch in Mischungen eingesetzt werden. Besonders günstige Kombinationen bestehen aus den Derivaten des Benzoylmethans, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert. Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Dimethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal 122, 543 (1996**)** sowie Parfümerie und Kosmetik 3 (1999), Seite 11ff zu entnehmen.

UVA-Strahlen dringen bis in die Dermis ein, wo sie zu Oxidationsstreß führen, was durch eine Lipoperoxidation der Zytoplasmamembranen nachgewiesen wird. Die Lipoperoxide werden zu Malonaldialdehyd abgebaut, der viele biologische Moleküle wie Proteine und Nukleinbasen vernetzen wird (Enzymhemmung bzw. Mutagenese). Glutathione (GSH) ist ein Peptid, welches direkt von den Zellen produziert wird um oxidativern Stress oder schädigenden Umwelteinflüssen wie zum Beispiel einer erhöhten Quecksilber- oder Bleibelastung entgegen zu wirken. Der verbleibende Gehalt an GSH nach der Bestrahlung mit UVA-Strahlung wurde bestimmt nach der Methode von Hissin, beschrieben in Anal. Biochem., 74, 214-226,1976.

UVB-Strahlen lösen durch Aktivierung eines Enzyms, nämlich Phospholipase A2 oder PLA2 eine Entzündung aus. Diese Entzündung (Erythem, Ödem) wird durch die Entfernung von Arachidonsäure aus den Phospholipiden der Plasmamembran durch die Phospholipase ausgelöst. Arachidonsäure ist die Vorstufe der Prostaglandine, die eine Entzündung und eine Zellmembranschädigung verursachen; die Prostaglandine E2 (= PGE2) werden durch die Cyclooxygenase gebildet. Der Grad der Freisetzung des Cytoplasmaenzyms LDH (Lactat Dehydrogenase) in humanen Keratinocyten dient als Marker für eine Zellschädigung.

Die erfindungsgemäßen Extrakte der Pflanze Cassia alata reduzieren den Effekt von UVB-Strahlung auf die Anzahl an Keratinocyten und auf den Gehalt an freigesetzte LDH. Die Extrakte zeigen demnach die Fähigkeit, die durch UVB-Strahlung hervorgerufene Schädigung an Zellmembranen zu reduzieren.

Die Verwendung der erfindungsgemäßen Extrakte als anti-inflammatorische Additive ist prinzipiell für alle kosmetischen und/oder dermatologischen Pflegemittel möglich, die bei Entzündungen der Haut und damit in der Hautpflege eingesetzt werden. Als anti-inflammatorisches Pflegemittel sind im Sinne der Erfindung die Art der Pflegemittel zu verstehen, die eine Entzündung der Haut heilen können oder die einer Entzündung vorbeugen können. Die Entzündungen können dabei die unterschiedlichsten Ursachen aufweisen.

Als Antioxidantien im Sinne der Erfindung sind Oxidationsinhibitoren zu verstehen, die sich aus der Pflanze Cassia alata isolieren lassen. Antioxidantien sind in der Lage, die unerwünschten, durch Sauerstoff-Einwirkungen und andere oxidative Prozesse bedingten Veränderungen in den zu schützenden Stoffen zu hemmen oder zu verhindern. Die Wirkung der Antioxidantien besteht meist darin, dass sie als Radikalfänger für die bei der Autoxidation auftretenden freien Radikale wirken.

Neben der Verwendung von Extrakten der Pflanze Cassia alata als Antioxidantien können auch weitere, bereits bekannte Antioxidantien eingesetzt werden. Eine mögliche Anwendung der Antioxidantien zum Beispiel in kosmetischen Zubereitungen, ist die Anwendung als sekundäre Lichtschutzmittel, weil Antioxidantien in der Lage sind, die photochemische Reaktionskette zu unterbrechen, welche ausgelöst wird, wenn UV-Strahlung in die Haut eindringt. Neben dem erfindungsgemäßen Pflanzenextrakt sind weitere typische Beispiele hierfür Aminosäuren (z.B. Glycin, Alanin, Arginin, Serin, Threonin, Histidin, Tyrosin, Tryptophan) und deren Derivate, Imidazole (z.B. Urocaninsäure) und deren Derivate, Peptide wie D,L-Camosin, D-Carnosin, L-Camosin und deren Derivate (z.B. Anserin), Carotinoide, Carotine (z.B. α-Carotin, β-Carotin, Lycopin, Lutein) oder deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystein, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, Boldin, Boldo-Extrakt, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stillbenoxid) und die erfindungsgemäß geeigneten Derivate (Salze, Ester, Ether, Zucker, Nukleotide, Nukleoside, Peptide und Lipide) dieser genannten Wirkstoffe.

Die UV-Lichtschutzfaktoren bzw. Antioxidantien, die neben den Extrakten aus Cassia alata eingesetzt werden, können in Mengen von 0,01 bis 25, vorzugsweise 0,03 bis 10 und insbesondere 0,1 bis 5 Gew.-% bezogen auf die Gesamtmenge in den Zubereitungen, zugegeben werden.

Die revitalisierende und reaktivierende Aktivität von Extrakten der Pflanze Cassia alata wirkt der Apoptosis entgegen. Die Verwendung der erfindungsgemäßen Extrakte als schützende und aufbauende Pflegemittel ist prinzipiell für alle Zubereitungen möglich, die zur Prävention gegen Schädigungen oder bei Schädigungen der Haut und damit in der Hautpflege eingesetzt werden. Eine andere Verwendung auf diesem Gebiet ist die Applikation bei empfindlicher, durch Allergie oder anderen Ursachen geschädigter Haut. Die Schädigung der Haut kann dabei unterschiedlichste Ursachen haben.

### Dermale Makromoleküle

Als dermale Makromoleküle sind im Sinne der Erfindung prinzipiell alle Makromoleküle zu verstehen, die als Bestandteile der Haut entweder in der Basalmembran zwischen Dermis und Epidermis oder in der Dermis und Epidermis direkt zu finden sind. Es handelt sich bei den dermalen Makromolekülen im Besonderen um solche, die ausgewählt sind aus der Gruppe, die gebildet wird aus Glykosaminoglykane insbesondere Chondroitinsulfat, Keratansulfat, Dermatansulfat und Hyaluronsäure und deren Salze, Collagen insbesondere Collagen Typ III, Elastin, Fibronectin, Proteoglycanen und deren Salze.

Die **Glykosaminoglykane** werden auch als Mucopolysaccharide bezeichnet. Es handelt sich um negativ geladene, lange unverzweigte Polysacharide (Glykane), welche aus 1,4-verknüpften Einheiten von Dissachariden bestehen, in denen ein Mol einer Uronsäure (D-Glucuronsäure oder beispielsweise L-Iduronsäure) mit der 3-Stellung eines N-acetylierten Aminozuckers (Glykosamins) glykosisdisch verbunden sind. Die Glykosaminoglykane sind im Gewebe zu mehreren Ketten an ein Kem-Protein (core protein) gebunden und bilden so die Proteoglykane. Das **Chondrotinsulfat** zählt zu den Glykosaminoglykanen. Es kommt im Gewebe als Chondroitin-4-sulfat oder als Chondroitin-6-sulfat vor und besteht u.a aus D-Glucuronsäure und N-Acetyl-D-galactosamin. Die molare Masse beträgt zwischen 5000-50000. Das auch als beta-Heparin bezeichnete, nicht gerinnungshemmend wirksame Glycosaminoglykan Dermatansulfat besteht aus L-Iduronsäure oder D- Glucuronsäure, N-Acetyl-D-galactosamin und Sulfat-Gruppen. Die molare Masse von Deramtansulfat liegt zwischen 15000 und 40000. Die Hyaluronsäure ist ein saures Glykosaminoglykan, Grundbaustein der Hyaluronsäure ist ein aus D-Glucuronsäure und N-Acetyl-D-glucosamin in (beta 1-3)-glykosidischer Bindung aufgebautes Aminodisaccharid, das mit der nächsten Einheit (beta 1-4)-glykosidisch verbunden ist. Die Hyaluronsäure trägt im Gegensatz zu vielen anderen Glykosaminoglykanen keine Sulfatgruppen und ist nicht proteingebunden im Gewebe.

Collagen besteht aus Proteinfasem und kommt in menschlicher Haut in drei verschiedenen Typen (Typ I, III und IV) vor. Im Collagen sind die einzelnen Polypeptidketten, die jeweils viel von der Aminosäure Prolin und als jeden dritten Rest Glycin enthalten, umeinander zu einer Tripelhelix gewunden. Die Collagenfasem werden als Tropokollagen in den Fibroblasten synthetisiert und in die extrazelluläre Matrix ausgeschleust. Die erfindungsgemäße Stimulierung der Synthese des Collagens führt zu einer Erhöhung der Produktion an Collagen und damit zu einer erhöhten intermolekularen Verfestigung der Dermis und dadurch zu einer straffer erscheinenden Haut. Das Elastin ist ebenfalls ein faserartiges Protein. Hierbei handelt es sich um unstrukturierte kovalent quervernetzte Polypeptidketten, die ein gummiähnliches elastisches Material bilden. Das Elastin wird nach der Synthese in den Hautzellen in die extrazelluläre Matrix ausgeschleust. Die erfindungsgemäße Stimulierung der Synthese der Elastin-Polypeptidketten führt zu einer Erhöhung der Produktion an Elastin und damit zu einer Erhöhung der Elastizität der Haut.

Fibronectin stellt eine Gruppe hochmolekularer Glykoproteine (MR des Dimers ca. 440 000-550 000) dar, die sich in der extrazellulären Matrix und in extrazellulären Flüssigkeiten finden. Das durch zwei Disulfid-Brücken verbundene Fibronectin-Dimer, ein langgestrecktes Molekül mit den Abmessungen 600×25 Å, bindet durch lineare Kombination dreier verschiedener sich wiederholender Domänen u. a. Collagene, Glykosaminoglykane, Proteoglykane, Fibrin(ogen), Desoxyribonucleinsäuren, Immunglobuline, Plasminogen, Plasminogen-Aktivator, Thrombospondin, Zellen und Mikroorganismen. Durch diese Eigenschaften vermittelt es z. B. die Anhaftung von Bindegewebszellen an Collagen-Fibrillen oder von Thrombocyten und Fibroblasten an Fibrin (Beitrag zur Wundheilung).

Die **Proteoglycane** bestehen wie die Glycoproteine aus Kohlenhydraten und aus Proteinen, bei den Proteoglycanen überwiegt jedoch der Anteil an Polysacchariden. Die Proteoglycane der Haut enthalten Dermatansulfat. Es lagern sich ca. 140 solcher Proteoglycane mit Hilfe kleinerer Proteine (Link-Proteine) nichtkovalent an eine Hyaluronsäure-Kette zu Molekül-Aggregaten mit einer mittleren Molmasse von ca. 2 Mio. an. Die durch ihr Wasserbindevermögen ausgezeichneten polyanionischen Aggregate können feste Gele bilden, die dem Stützgewebe (extrazelluläre Matrix) Elastizität und Zugfestigkeit verleihen. In Schleimen schützen sie die Epithelien. Die erfindungsgemäße Stimulierung der Synthese von Proteoglycanen und Hyaluronsäure führt zu einer größeren Menge an extrazellulärer Matrix und damit zu einer erhöhten Elastizität und Zugfestigkeit.

Bei der Proteolyse handelt es sich um einen Prozess, bei dem es zu einer Spaltung von Proteinen durch Hydrolyse der Peptid-Bindungen mittels Säuren oder Enzymen kommt. Eine andere Bezeichnung ist die Proteinase Verdauung. Die erfindungsgemäße Verminderung der Proteolyse führt zu einer verminderten Spaltung der dermalen Makromoleküle, die eine Proteinstruktur aufweisen und damit zur Verhinderung der Verminderung einer Hautfestigung und zur Verhinderung des Abbaus einer erhöhten Elastizität. Die erfindungsgemäßen Extrakte aus Cassia alata wirken als Proteaseinhibierendes Mittel, insbesondere als MMP- und/oder Collagenase- und/oder Elastase-inhibierendes Mittel. Unter **MMP** versteht man Matrix-Metallo-Proteasen. Zu den Matrix-Metallo-Proteasen zählen u.a. Collagenase, aber auch eine bestimmte Art der Elastasen. Die Aktivität der Enzyme ist abhängig von Metallionen - häufig handelt es sich um Zn²⁺-Ionen. Die hauptsächlich vorkommende Elastase zählt zur Gruppe der Serin-Proteasen. Ihre katalytische Reaktion beruht auf einen anderen Mechanismus. Diese Proteasen (Collagenase und die unterschiedlichen Elastasen) katalysieren die Fragmentierung und Zerstörung der dermalen Makromoleküle wie Proteoglycan, Collagen und Elastin und führen dadurch zur Alterung der Haut und zu den Effekten der natürlichen Hautalterung nach UV-Strahlung.

Die Glycation ist eine nichtenzymatische Reaktion von Glucose oder anderen Zuckern mit Proteinen zu Glycoproteinen. Diese Reaktion führt zu unbeabsichtigten Veränderungen am Collagen und Elastin und damit zu Veränderungen der extrazellulären Matrix. Die Funktion des Collagens und der extrazellulären Matrix sind gestört. Die erfindungsgemäße Verhinderung der Glycation führt zu einer Verringerung der nichtenzymatischen Veränderung an Collagen und Elastin und damit zur Verhinderung einer verminderten Funktion der extrazellulären Matrix.

Die erfindungsgemäßen Zubereitungen können zur Herstellung von kosmetischen und/oder dermatologischen Zubereitungen, wie beispielsweise Schaumbäder, Duschbäder, Cremes, Gele, Lotionen, alkoholische und wäßrig/alkoholische Lösungen, Emulsionen, Wachs/ Fett-Massen, Stiftpräparaten, Pudern oder Salben zum Einsatz kommen.

Diese Zubereitungen können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, biogene Wirkstoffe, Deodorantien, Antitranspirantien, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin, 1987, S. 54-124 **oder** J.Falbe (ed.), "Katalysatoren, Tenside und Mineralöladditive", Thieme Verlag, Stuttgart, 1978, S. 123-217 verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. DE 19756377 A1), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8
bis 15 C-Atomen in der Alkylgruppe sowie Alkylamine mit 8 bis 22 Kohlenstoffatomen im Alkylrest; ➢ Alkyl- und/oder Alkenyloligoglykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl; ➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 **PS** und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Polymeremulgatoren, z.B. Pemulen-Typen (TR-1,TR-2) von Goodrich;
➢ Polyalkylenglycole sowie
➢ Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE 2024051 **PS** als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispiels-weise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvemetzte und mit Polyolen vernetzte Polyacrylsäuren, Acrylamidopropyltrimethylammoniumchlorid/Acrylat-Copolymere, Octylacrylamid/Methylmeth-acrylat/tertButylaminoethylmethacrylat/2-Hydroxypropylmethacrylat-Copolymere, Polyvinylpyrrolidon, VinylpyrrolidonNinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in **Cosm.Toil.108, 95 (1993)** aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in **Cosm.Toil. 91, 27 (1976)**.

### Biogene Wirkstoffe

Unter biogenen Wirkstoffen sind im Rahmen der Erfindung zusätzlich solche zu verstehen, die nicht aus der Pflanze Cassia alata stammen, wie beispielsweise Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Aminosäuren, Ceramide, Pseudoceramide, essentielle Öle, weitere Pflanzenextrakte und zusätzliche Vitaminkomplexe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren. Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)harnstoff, 2,4,4'-Trichlor-2'-hydroxydiphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren,
➢ Coemulgatoren,
➢ Konsistenzgeber,
➢ Hilfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-nium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toif.108, 95** (**1993**) entnommen werden.

### Insekten-Repellentien

Als Insekten-Repellentien kommen N,N-Diethyl-m-toluamid, 1,2-Pentandiol oder Ethyl Butylacetylaminopropionate in Frage

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope,

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglykol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methyolverbindungen, wie insbesondere Trimethylolethan, Trimethylolpropan, Trimethylolbutan, Pentaerythrit und Dipentaerythrit;
➢ Niedrigalkylglucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronen, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

### Beispiele

### 1. Beispiel: Extraktion der Pflanzen mit destilliertem Wasser

100 g getrocknete Blätter der Pflanze Cassia alata wurden in einer mit Messern versehenen Zerkleinerrungsmaschine grob zerkleinert und dann in einen Glasreaktor mit 1 l destilliertem Wasser überführt. Der Aufguß wurde zwischen 85 und 90 °C erhitzt und unter Rühren über einen Zeitraum von 1 h bei dieser Temperatur extrahiert. Anschließend wurde die Mischung auf 20 °C abgekühlt und 20 min bei einer Geschwindigkeit von 5000 g zentrifugiert. Die überstehende Flüssigkeit wurde durch Filtration an Tiefenfilter mit einer mittleren Porosität von 450 nm (von der Firma Seitz, Bordeaux Frankreich) vom unlöslichen Rückstand getrennt. Der Extrakt war braun gefärbt. Der Extrakt wurde bei einer Anfangstemperatur von 185 °C und einer Endtemperatur von 80 °C sprühgetrocknet. Eine weitere Möglichkeit der Trocknung des Extraktes ist die Lyophilisierung. Es wurden Pflanzen aus drei unterschiedlichen Ländern (Ghana, Indien und Benin) extrahiert. Die Ausbeute an Trockenprodukt betrug 12,4 bis 18,7 Gew.-% bezogen auf das Trockengewicht an eingesetzten Pflanzen.

**Tabelle 1: Ausbeuten an Trockenprodukt der extrahierten Pflanze nach der Extrakten mit destilliertem Wasser**

| **Herkunftsland** | **Batch** | **Auseute (Gew.-%)** | **Trockenmethode** |
|---|---|---|---|
| Ghana | A | 13,5 | Lyophilisieren |
| Indien | B | 18,7 | Spraytrocknung |
| Benin | C | 12,4 | Spraytrocknung |

### 2. Beispiel: Extraktion der Pflanzen mit wässrigem Methanol

Beispiel 1 wurde wiederholt, die Extraktion jedoch mit 1 l 50 Gew.-%-igem wässrigen Methanol durchgeführt. Die Extraktion wurde unter Rühren 1 h bei einer Temperatur zwischen 80 und 85 °C durchgeführt und der Extrakt wie beschrieben weiter verarbeitet. Die Filtration wurde wie im Beispiel 1 beschrieben durchgeführt. Anschließend wurde zunächst der Alkohol bei 35 °C unter vermindertem Druck entfernt und dann der braune Rückstand wie beschrieben sprühgetrocknet bzw. wahlweise lyophilisiert. Die Ausbeute an Trockenprodukt betrug 15,1 bis 18,4 Gew.-% bezogen auf das Trockengewicht an eingesetzten Pflanzen.

**Tabelle 2: Ausbeuten an Trockenprodukt der extrahierten Pflanze nach Extraktion mit 50%-igem wässrigen Methanol**

| **Herkunftsland** | **Batch** | **Ausbeute (Gew.-%)** | **Trockenmethode** |
|---|---|---|---|
| Ghana | A | 18,4 | Lyophilisieren |
| Indien | B | 15,1 | Spraytrocknung |
| Benin | C | 15,2 | Spraytrocknung |

### 3. Beispiel: Antioxidative und radikalfangende Eigenschaften

In einer ersten Testreihe wurde die Eignung der Extrakte gegen oxidativen Stress untersucht. Eingesetzt wurden die Extrakte gemäß der Beispiele 1 und 2 jeweils in unterschiedlichen Konzentrationen. In einem ersten Test wurde als Referenzsystem die Hydroxylierung von Salicylsäure durch Hydroxylradikale (aus der Reaktion von Wasserstoffperoxid mit Eisen(III)ionen und EDTA) untersucht. Diese Reaktion kann photometrisch untersucht werden, da das Hydroxylierungsprodukt der Salicylsäure rötlich gefärbt ist. Gemessen wurde der Einfluss der Extrakte auf die Bildung der Hydroxysalicylsäure bei einer optischen Dichte von 490 nm. Die Meßergebnisse sind in Tabelle 1 zusammengefaßt, angegeben ist die Konzentration in w/v (weight per volume) an Extrakt aus Cassia alata die zu einer 50 %-igen Inhibierung (IC₅₀ % w/v) der Hydroxylierung notwendig ist.

**Tabelle 3: Konzentration an Extrakt für eine 50 % ige Inhibierung der Hydroxylierung**

| | Extrakt nach Beispiel 1 | | | Extrakt nach Beispiel 2 | | |
|---|---|---|---|---|---|---|
| Batch | A | B | C | A | B | C |
| IC₅₀ % w/v | 0,18 | 0,06 | 0,07 | 0,33 | 0,10 | 0,12 |

Aus den Werten der Tabelle 3 lässt sich erkennen, dass die verwendeten Extrakte der Pflanze Cassia alata eine Wirkung gegen Radikale zeigen. Je nach Extraktionsverfahren erhält man unterschiedlich wirksame Extrakte. Nach einem Extraktionsverfahren beschrieben in Beispiel 1 ist z.B. eine Konzentration von 0,06 % w/v ausreichend um eine 50 %-ige Inhibierung der Radikalreaktion zu erzielen. Die Bildung von Hydroxysalicylsäure durch Hydroxyradikale ist bei dieser Konzentration in diesem Fall um 50 % reduziert.

In einem dritten Test wurde Xanthin Oxidase als Testsystem gewählt. Das Enzym bewirkt bei oxidativem Stress die Umwandlung von Purinbasen, wie z.B. Adenin oder Guanin in Harnsäure, wobei die intermediär gebildeten Sauerstoffradikale durch Reaktion mit Luminol über die Lumineszenz nachgewiesen und quantitativ bestimmt werden können. In Gegenwart von Substanzen mit radikalfangenden Eigenschaften vermindert sich die Lumineszenzausbeute.

**Tabelle 4: Grad der Lumineszenzhemmung**

| | Extrakt nach Beispiel 1 | | | Extrakt nach Beispiel 2 | | |
|---|---|---|---|---|---|---|
| Batch | A | B | C | A | B | C |
| IC₅₀ % w/v | 0,015 | 0,007 | 0,007 | 0,009 | 0,006 | 0,005 |

Aus der Tabelle 4 lässt sich entnehmen, dass die Extrakte der Pflanze Cassia alata die radikalische Bildung von Lumineszens inhibieren. Eine Konzentration von 0,005 % w/v eines Extraktes hergestellt nach Beispiel 2 liefert bereits eine 50 %-ige Inhibierung der Lumineszenzbildung und zeigt damit deutlich radikalfangende Eigenschaften.

### 4. Beispiel: Inhibierung der Apoptose-Induktion

Hintergrund: Die Apoptose ist im Gegensatz zur Nekrose ein natürlicher gezielter Zelltod bestimmter unerwünschter oder geschädigter Zellen. Es handelt sich um einen aktiven Prozess der Zellen (Selbstmord auf Befehl). Apoptose kann durch einen oxidativen Stress (UV-Strahlung, Entzündung), durch einen Mangel an Wachstumsfaktoren oder durch giftige Stoffe (Schmutzstoffe, genotoxische Stoffe usw.) eingeleitet werden. Bei der Hautalterung z. B. kann es durch einen Mangel an Wachstumsfaktoren in der Haut zu einer induzierten Apoptose der Hautzellen kommen. Bei den durch Apoptose betroffenen Zellen wird durch das spezifische Enzym Endonuclease die nukleare DNA abgebaut und die DNA-Fragmente in das Cytoplasma geschleust.

Methode: Untersucht wurde die Fähigkeit der Pflanzenextrakte aus Cassia alata, die durch einen Mangel an Wachstumsfaktoren induzierte Apoptose in humanen Hautzellen zu verhindern. An humanen Fibroblasten und humanen Keratinocyten wurden diese Test in vitro durchgeführt. Die humanen Zellen wurden in einem Nährmedium (DMEM = Dulbecco Minimum Essential Medium von der Firma Life Technologie Sarl) mit 10 % fötalem Kälberserum (von der Firma Dutcher) kultiviert. Diesem Nährmedium wurde Bromodesoxyuridin (BrdU) zugegeben, welches in die DNA eingebaut wurde und später zum Nachweis der DNA-Fragmente in dem Cytoplasma diente. Nach zwei Tagen Inkubationsdauer wurde das Nährmedium gegen Nährmedium (DMEM) ohne fötalem Kälberserum ausgetauscht. Die zu testende Aktivsubstanz wurde zugegeben. Für den Pflanzenextrakt wurden drei unterschiedliche Batch d.h. zwei unterschiedliche Extrakte (Batch A, B und C) der gleichen Extraktionsmethode getestet. Zum Vergleich wurde eine Zellprobe ohne zu testende Aktivsubstanz (Mengen und Konzentration angegeben in der Tabelle 5 und 6) inkubiert.

Nach einer weiteren Inkubationszeit von einem oder zwei Tagen bei 37 °C wurden die Zellen durch Trypsination zurückgewonnen nach der Methode von Dunnebacke und Zitcer beschrieben in: Cell and tissue culture, Hrsg.: J. Paul, Churchill Livingstone, 1975, S. 226. Nach der Trypsinierung wurden die Zellen zentrifugiert und ausgezählt. Anschließend wurde der Gehalt an BrdU in DNA-Fragmenten aus dem Cytoplasma mit Hilfe des ELISA Tests (ELISA Kit der Firma Roche) ermittelt. Der Gehalt an BrdU ist ein Maß für die DNA-Fragmente, die aus dem Nukleus, dem Zellkern, in das Cytoplasma geschleust wurden. Die Ergebnisse wurden auf eine Millionen Zellen bezogen und in Prozent im Vergleich zur Kontrolle angegeben. Die Ergebnisse sind in den folgenden Tabellen zusammengefasst.

**Tabelle 5: Anzahl der Zellen und Gehalt an DNA-Fragmenten im Cytoplasma nach der Behandlung von humanen Fibroblasten mit Extrakten von Cassia alata.**

| **Zellzählung** | **Batch** | | | **Gehalt an DNA-Fragmenten** | **Batch** | | |
|---|---|---|---|---|---|---|---|
| | **A** | **B** | **C** | | **A** | **B** | **C** |
| Kontrolle | 100 | 100 | 100 | Kontrolle | 100 | 100 | 100 |
| Extrakt nach Beispiel **2;** 0,01 Gew-% | 104 | 106 | 149 | Extrakt nach Beispiel **2;** 0,01 Gew-% | 23 | 85 | 94 |
| Extrakt nach Beispiel **2;** 0,02 Gew-% | 122 | 112 | 146 | Extrakt nach Beispiel **2;** 0,02 Gew-% | 11 | 76 | 59 |

**Tabelle 6: Anzahl der Zellen und Gehalt an DNA-Fragmenten im Cytoplasma nach der Behandlung von humanen Keratinocyten mit Extrakten von Cassia alata**

| **Zellzählung** | **Batch** | | | **Gehalt an DNA-Flagmenten** | **Batch** | | |
|---|---|---|---|---|---|---|---|
| | **A** | **B** | **C** | | **A** | **B** | **C** |
| Kontrolle | 100 | 100 | 100 | Kontrolle | 100 | 100 | 100 |
| EGF 30 ng/ml | 121 | | | EGF 30 ng/ml | 32 | | |
| Extrakt nach Beispiel **1;** 0,01 Gew-% | | 105 | | Extrakt nach Beispiel **1;** 0,01 Gew-% | | 63 | |
| Extrakt nach Beispiel **1;** 0,02 Gew.-% | 105 | | 101 | Extrakt nach Beispiel **1;** 0,02 Gew-% | 39 | | 63 |
| Extrakt nach Beispiel **1;** 0,05 Gew-% | 108 | 105 | 103 | Extrakt nach Beispiel **1;** 0,05 Gew-% | 25 | 47 | 48 |
| | | | | | | | |

| **Zellzählung** | **Batch** | | | **Gehalt an DNA-Fragmenten** | **Batch** | | |
|---|---|---|---|---|---|---|---|
| | **A** | **B** | **C** | | **A** | **B** | **C** |
| Kontrolle | 100 | 100 | 100 | Kontrolle | 100 | 100 | 100 |
| Extrakt nach Beispiel **2;** 0,01 Gew-% | 104 | 106 | 149 | Extrakt nach Beispiel **2;** 0,01 Gew-% | 23 | 85 | 94 |
| Extrakt nach Beispiel **2;** 0,02 Gew-%- | 122 | 112 | 146 | Extrakt nach Beispiel **2**; 0,02 Gew-% | 11 | 76 | 59 |

Aus den Ergebnissen, dargestellt in den Tabellen 5 und 6, lässt sich erkennen, dass durch den Einsatz von Extrakten aus der Pflanze Cassia alata die Apoptose in humanen Zellkulturen in vitro abnimmt. Der Gehalt an freien DNA-Fragmenten im Cytoplasma und damit der Grad an zerstörter DNA im Zellkern und der Grad an Apoptose nimmt mit zunehmender Konzentration an Pflanzenextrakt aus Cassia alata ab. Im Vergleich zu dem bekannten Wachstumsfaktor Epidermal growth factor (EGF), der in einer Konzentration von 30 ng/ml zugesetzt wurde anstelle des Pflanzenextraktes, zeigt sich für den Pflanzenextrakt eine ebenso gute Eigenschaft in Bezug auf die Reduzierung der Apoptose. Die Werte der Zellzählung dokumentieren, dass die erfindungsgemäßen Pflanzenextrakte nicht toxisch sind und nicht zum Zelltod führen. Die Anzahl der vorhandenen intakten Zellen ist kaum verändert, der Gehalt an DNA-Fragmenten im Cytoplasma ist unter Einfluss des Pflanzenextraktes im Vergleich zur Kontrolle jedoch verringert. Der gezielte Zelltod wird durch diese Pflanzenextrakte unterdrückt. Diese Pflanzenextrakte zeigen einen Wachstumsfaktor ähnlichen Effekt und damit einen "anti-ageing" Effekt an humanen Hautzellen.

### 5. Beispiel: Entzündungshemmende Eigenschaften in vitro - UVB Lichtschutz

Hintergrund: UVB-Strahlen lösen durch Aktivierung eines Enzyms, nämlich Phospholipase A2 oder PLA2, die Arachidonsäure aus den Phospholipiden der Zellmembran entfernt, eine Entzündung (Erythem, Ödem) aus. Arachidonsäure ist die Vorstufe der Prostaglandine, die eine Entzündung und eine Zellmembranschädigung verursachen; die Prostaglandine E2 (= PGE2) werden durch die Cyclooxygenase gebildet.

Methode: Der Effekt von UVB-Strahlung wurde an Keratinocyten in vitro untersucht indem die Freisetzung des Cytoplasaenzyms LDH (Lactat Dehydrogenase) bestimmt wurde. Dieses Enzym dient als Marker für eine Zellschädigung.

Zur Durchführung der Tests wurde ein definiertes Medium, das fötales Kälberserum enthält, mit den Keratinozyten beimpft und der Pflanzenextrakt 72 Stunden nach dem Beimpfen zugegeben.

Die Keratinozyten wurden sodann mit einer UVB-Dosis bestrahlt (50 mJ/cm² - Röhren: DUKE GL40E).

Nach weiterer 1 tägiger Inkubation bei 37 °C und bei 5 % CO₂ wurde der LDH- und der PGE2-Gehalt im Überstand bestimmt. Der Gehalt von LDH- (Lactatdehydrogenase) wurde mittels einer Enzymreaktion bestimmt (verwendetes kit zur Untersuchung des LDH Gehaltes von der Firma Roche) Der Gehalt an PGE2 wurde mit einem ELISA-Test (ELISA Kit der Firma Roche) bestimmt. Zur Bestimmung des DNA Anteils im Cytoplasma der Keratinocyten wurde wie bereits im vorherigen Beispiel beschrieben, dem Wachstumsmedium Bromodesoxyuridine (BrDU) zugegeben. Nach der Trypsin-Behandlung wurden die Zellen zentrifugiert und ausgezählt. Anschließend wurde der Gehalt an BrdU in DNA-Fragmenten aus dem Cytoplasma mit Hilfe des ELISA Tests ermittelt. Die Anzahl adhärenter Keratinozyten wird (nach Trypsinbehandlung) mit einem Partikelzählgerät bestimmt.

**Tabelle 7: Zellschutzwirkung eines Extraktes von Cassia alata gegen UVB-Strahlen; Ergebnisse in % bezogen auf die Kontrolle, Mittelwert aus 2 Versuchen, jeder mit zwei Wiederholungen**

| **Extrakt nach Beispiel 1** | **Anzahl Keratinocyten** | | | **Gehalt an freigesetzte LDH** | | |
|---|---|---|---|---|---|---|
| | **A** | **B** | **C** | **A** | **B** | **C** |
| Kontrolle ohne UV | 331 | 195 | 200 | 0 | 0 | 0 |
| Kontrolle mit UVB (315 nm) | 100 | 100 | 100 | 100 | 100 | 100 |
| UVB + Extrakt 0,01 % | 109 | 114 | 282 | 87 | 131 | 0 |
| UVA + Extrakt 0,05 % | 189 | 118 | 295 | 24 | 82 | 0 |
| | | | | | | |

| **Extrakt nach Beispiel 1** | **Gehalt an freigesetztem PGE2** | | | **Gehalt an DNA Fragmenten** | | |
|---|---|---|---|---|---|---|
| | **A** | **B** | **C** | **B** | | **C** |
| Kontrolle ohne UV | 0 | 0 | 0 | 0 | | 0 |
| Kontrolle mit UVB (315 nm) | 100 | 100 | 100 | 100 | | 100 |
| UVB + Extrakt 0,01 % | 112 | 75 | 0 | - | | 57 |
| UVA + Extrakt 0,05% | 40 | 91 | 0 | 27 | | 9 |
| | | | | | | |

| **Extrakt nach Beispiel 2** | **Anzahl keratinocyten** | | | **Gehatt an freigesetzte LDH** | | |
|---|---|---|---|---|---|---|
| | **A** | **B** | **C** | **A** | **B** | **C** |
| Kontrolle ohne UV | 331 | 173 | 188 | 0 | 0 | 0 |
| Kontrolle mit UVB (315 nm) | 100 | 100 | 100 | 100 | 100 | 100 |
| UVB + Extrakt 0,01 % | 258 | 178 | - | 14 | 25 | - |
| UVB + Extrakt 0,02 % | 369 | 220 | 337 | 19 | 10 | 2 |
| UVA + Extrakt 0,05 % | - | 226 | 354 | - | 18 | 7 |
| | | | | | | |

| **Extrakt nach Beispiel 2** | **Gehalt an freigesetztem PGE2** | | | **Gehalt an DNA Fragmenten** | | |
|---|---|---|---|---|---|---|
| | **A** | **B** | **C** | **B** | | **C** |
| Kontrolle ohne UV | 0 | 0 | 0 | 0 | | 0 |
| Kontrolle mit UVB (315 nm) | 100 | 100 | 100 | 100 | | 100 |
| UVB + Extrakt 0,01 % | 35 | 208 | - | - | | 63 |
| UVB + Extrakt 0,02 % | 19 | 78 | 12 | 55 | | 62 |
| UVA + Extrakt 0,05 % | - | - | 14 | 44 | | 90 |

Die Ergebnisse dieser Tests belegen, dass ein erfindungsgemäßer Extrakt der Pflanze Cassia alata den Effekt von UVB-Strahlung auf die Anzahl an Keratinocyten reduziert. Es zeigt sich eine Verringerung des durch UVB an menschlichen Keratinocyten induzierten PGE2 - Gehaltes, eine Verringerung des Gehalts an freigesetzte LDH und eine Reduzierung an DNA Fragmenten im Cytoplasma. Die beschriebenen Extrakte zeigen demnach die Fähigkeit, die durch UVB-Strahlung hervorgerufene Schädigung an Zellmembranen zu reduzieren und zeigen eine hemmende Wirkung gegen Entzündungen, die durch UVB Strahlung induziert werden.

### 6. Beispiel: Nachweis der Stimulierung der Synthese von dermalen Makromolekülen (GAG)

Hintergrund: Das Ziel dieser Untersuchungen ist der Nachweis einer stimulierenden Aktivität von Extrakten aus Cassia alata auf die Synthese von dermalen Makromolekülen an humanen Fibroblastenkulturen in vitro.

Die Dermis ist aufgebaut aus Zellen (Fibroblasten und Mastzellen), Gewebebestandteilen (Collagen und Elastin) und aus sogenannten Grundsubstanzen. Zu diesen Grundsubstanzen zählen beispielsweise Glykosaminoglykan (GAG), Hyaluronsäure, Chondroitinsulfat, Dermatansulfat und Glycoproteine. Durch die Hautalterung vermindert sich die intermolekulare Verfestigung und Elastizität der Dermis und dadurch die Straffheit der Haut. Ebenso wird die Zahl der vorhandenen Hautzellen, insbesondere der Fibroblasten im Laufe der Hautalterung reduziert. Die Collagenfasern werden im Laufe der Zeit fragmentiert und es erhöht sich der Anteil von unlöslichen zu löslichen Collagen. Die feinen dermalen elastischen Fasern vergröbern sich und werden zerstört. Die Synthese von GAG (Glycosaminoglycan) ist vermindert. All diese Prozesse tragen zur Hautalterung und deren Erscheinungsformen wie Falten und mangelnde Straffheit der Haut bei.

Mit folgendem Modell kann die Stimulierung der Synthese der dermalen Makromoleküle nachgewiesen werden und damit eine Aktivsubstanz identifiziert werden, die gegen Hautalterung wirken kann, also als anti-aging Mittel wirken kann.

Methode: Die Messmethode basiert auf eine Anfärbung von Makromolekülen in einer Kultur humaner Fibroblasten, die mit Collagen Typ I ein Collagen-Gel oder Collagen Gitterfasern aufbaut. Bestimmte Regionen dieser Fasern werden mit Hilfe von Anfärbereagenzien auf den Anteil an den genannten Makromolekülen quantifiziert.

Zu diesem Zweck vermischte man eine Suspension humaner Fibroblasten mit einer Lösung von Collagen Typ I (1-2 mg/ml). Diese Mischung wurde in einem definiertem Nährmedium (DMEM = Dulbecco Minimum Essential Medium, Firma Life Technologie Sarl) mit 0,5- oder 2 Gew.-% fötalem Kälberserum (FCS) bei 37 °C in einer 5 %igen CO₂-Atmosphäre in Petri-Schalen (5 ml pro Schale) unter Zusatz verschiedener Konzentrationen der zu untersuchenden Pflanzenextrakte über 14 Tage inkubiert.

Die Kinetik der Collagen-Gel Konzentration wurde 2 bis 3 mal pro Woche durch Messung von zwei perpendicularen Durchmessern an jedem Collagen-Gel mit einem Mikroskop mit Bildanalysesystem bestimmt. Die Größe der Fläche ist in der Tabelle 8 in cm² dargestellt. Nach 14-tägiger Inkubation wurde die Dichte des Collagen-Gels durch eine Bildanalyse mit einer Lichtquelle aus sichtbarem Licht bestimmt indem unterschiedliche Graustufen vergleichend untersucht wurden. Es handelt sich um eine relative Bestimmung der Dichte (0=klar oder weiss und 1=schwarz), die mit keiner Einheit versehen werden kann.

Nach sieben und nach 14 Tagen Inkubationszeit wurden Biopsien (Gewebeproben) genommen und histologische Schnitte des Collagen-Gels mit humanen Fibroblasten erhalten. Die Synthese von Makromolekülen wurde untersucht und durch die Anfärbung von Glycosaminoglycan mit PAS-Alcian blau z.B. von der Firma SIGMA nach der Periodic-Acid-Schiff-Methode (PAS), beschrieben in: Mowry RW, Anal. NY Adad. Sci. 106 Art 2, 402, 1963, quantifiziert. Die Beurteilung der Stimulierung der Synthese von Makromolekülen wurde direkt in der Umgebung von Fibroblasten durchgeführt. Diese Zone wird auch als "Perifibroblasten Fläche" bezeichnet.

Eine Quantifizierung der "Perifibroblasten" Sekretion bzw. der Sekretion von Fibroblasten in die Peripherie wurde durch ein Image-Analysator mittels eines Mikroskops durchgeführt. Detektiert wurden reaktive Strukturen in der "Perifibroblasten-Fläche" und die unterschiedlichen Graustufen vergleichend bestimmt. Dabei wurden die Werte der Graustufen unterteilt von O=weiss bis 255=schwarz. Diese Parameter sind direkt proportional zur Intensität der Synthese von Makromolekülen und damit zum GAG-Anteil der Fibroblasten. In der folgenden Tabelle sind die Ergebnisse der Werte dieser Parameter dargestellt und direkt als repräsentative Werte für die Syntheseaktivität der Fibroblasten zu sehen. Der Anteil der GAGs wird als relativer Wert der Graustufe beschrieben.

**Tabelle 8: Gehalt an GAGs in Gewebeproben humaner Fibroblasten mit Collagen nach der Behandlung mit Extrakt aus Cassia alata**

| **Extrakt nach Beipsiel 1** | **Flache [cm²]** | | **Dichte** | **Gags Anteil** | |
|---|---|---|---|---|---|
| | **7 Tage** | **14 Tage** | **14 Tage** | **7 Tage** | **14Tage** |
| FCS 0,5 Gew.-% | 7,6 | 4,4 | 0,36 | 12,25 | 10,76 |
| FCS 0,5 + Cassia alata 0,003 Gew.-% | 7,3 | 4,2 | 0,33 | 11,21 | 12,54 |
| FCS 0,5 + Cassia alata 0,01 Gew.-% | 10,3 | 5,9 | 0,27 | 11,09 | 15,36 |
| (FCS2Gew.-%) | 4,3 | 3,2 | 0,38 | 11,27 | 15,14 |
| | | | | | |

| **Extrakt nach Beispiel** | **Fläche [cm²]** | | **Dichte** | **GAGs Anteil** | |
|---|---|---|---|---|---|
| | **7 Tage** | **14 Tage** | **14 Tage** | **7 Tage** | **14Tage** |
| FCS 0,5 Gew.-% | 6,1 | 5,1 | - | 13,6 | - |
| FCS 0,5 + Cassia alata 0,003 Gew.-% | 11,5 | 5,3 | 0,28 | 17,1 | 19,5 |
| FCS 0,5 + Cassia alata 0,01 Gew.-% | 19,6 | 19,6 | 0,08 | - | - |
| FCS 2 Gew.-% | 3,0 | 2,5 | 0,45 | 14,9 | 15,0 |

Aus den Ergebnissen der Bestimmung des Glycosaminoglycan-Anteils in Gewebeproben von Collagen-Gel mit Fibroblasten speziell in der "perifibroblasten Fläche" lässt sich eine signifikante Erhöhung des GAG Anteils nach 14 tägiger Inkubationszeit mit unterschiedlichen Konzentrationen an Cassia alata Extrakt nach Beipsiel 1 im Vergleich zur Inkubation mit reinem Fötalem Kälber Serum (FCS) in einer Konzentration von 0,5 Gew.-% erkennen. Auch Extrakt nach Beispile 2 führt bereits bei einer Konzentration von 0,003 Gew.-% zu einer starken Erhöhung des GAG-Anteils. Diese Werte belegen, dass ein Extrakt der Pflanze Cassia alata die Synthese von Glycosaminoglycan (GAG) in Fibroblasten stimuliert.

Diese Ergebnisse belegen weiterhin, dass die Extrakte von Cassia alata eine hohe Kapazität zeigen um den Metabolismus von Fibroblasten anzuregen. Die Extrakte zeigen eine regenerierende und revitalisierende Aktivität an humanen Fibroblasten und sind damit als Energielieferanten und als anti-ageing Mittel in kosmetischen und in dermatologischen Zubereitungen einsetzbar.

### 7. Beispiel: Zellschutzwirkung gegen UVA an in vitro gezüchteten menschlichen Fibroblasten

Hintergrund: UVA-Strahlen dringen bis in die Dermis ein, wo sie zu Oxidationsstreß führen, was durch eine Lipoperoxidation der Zytoplasmamembranen nachgewiesen wird.

Die Lipoperoxide werden zu Malonaldialdehyd abgebaut, der viele biologische Moleküle wie Proteine und Nukleinbasen vernetzen wird (Enzymhemmung bzw. Mutagenese).

Glutathione (GSH) ist ein Peptid, welches direkt von den Zellen produziert wird um oxidativem Stress oder schädigenden Umwelteinflüssen wie zum Beispiel einer erhöhten Quecksilber- oder Bleibelastung entgegen zu wirken. Der verbleibende Gehalt an GSH nach der Bestrahlung mit UVA-Strahlung wurde bestimmt nach der Methode von Hissin, beschrieben in Anal. Biochem., 74, 214-226,1976.

Methode: Zur Durchführung dieser Tests wurde ein definiertes Kulturmedium (DMEM) mit 10 % fötalem Kälberserum mit den Fibroblasten beimpft und der Pflanzenextrakt (in dem definierten Medium mit 2% Serum) 72 Stunden nach dem Beimpfen zugegeben.

Nach 48 stündiger Inkubation bei 37 °C und einem CO₂-Gehalt von 5 % wurde das Kulturmedium durch eine Saline-lösung ersetzt und die Fibroblasten wurden mit einer UVA-Dosis bestrahlt (3 bis 15 J/cm²; Röhren: MAZDA FLUOR TFWN40).

Nach der Beendigung der Bestrahlung wurde der Gehalt an Zellproteinen und der Anteil an GSH bestimmt sowie der MDA-Spiegel (Malonaldialdehyd-Spiegel) in der überstehenden Saline-lösung quantitativ durch Reaktion mit Thiobarbitursäure bestimmt. Die Ergebnisse sind angegeben in Prozent im Vergleich zur Kontrolle ohne Bestrahlung.

**Tabelle 9 Quantifizierung von Malonaldialdehyd, in Fibroblasten (Ergebnisse in % bezogen auf die Kontrolle, Mittelwert aus 2 Versuchen, jeder mit drei Wiederholungen**

| Konzentration (Gew.-%) | MDA-Spiegel | | Gehalt an Zellproteinen | | Anteil an GSH | |
|---|---|---|---|---|---|---|
| Extrakte nach Beispiel 2 - Batch Nummer | C | B | C | B | C | B |
| Kontrolle ohne UV | 0 | 0 | 100 | 100 | 100 | 100 |
| UVA(3bis15J/cm²) | 100 | 100 | 93 | 81 | 78 | 94 |
| UVA + Extrakt 0,01 % | 42 | 81 | 100 | 121 | 114 | 88 |
| UVA + Extrakt 0,02 % | 35 | 63 | 98 | 120 | 137 | 87 |

Die Ergebnisse aus der Tabelle 9 zeigen, dass die erfindungsgemäßen Extrakte aus den Blättern der Pflanze Cassia alata signifikant den Grad an MDA in humanen Fibroblasten, welcher durch UVA-Strahlen induziert wird, reduzieren. Des weiteren ergibt sich eine hohe Aktivität für Batch C, den Anteil an GSH in humanen Fibroblasten nach einer Bestrahlung mit UVA-Strahlung relativ konstant zu halten. Diese Ergebnisse zeigen eine hohe Kapazität von Extrakten aus den Blättern von Argania spinosa schädliche Effekte eines oxidativen Stresses auf der Haut zu reduzieren.

### 8. Beispiel: Inhibierung der Elastase-Aktivität

Elastase ist eine Protease, welche entweder während einer Inflammation durch die Leukocyten oder infolge UV-A-Schädigung von den Fibroblasten ausgeschieden wird und für den Abbau von dermalen Makromolekülen, wie z.B. Kollagen und Elastin und damit für die Hautalterung mitverantwortlich ist. Zur Untersuchung der Wirksamkeit des Pflanzenextraktes die Freisetzung von Elastase zu inhibieren wurde Pankreaselastase (eine Serin-Protease) untersucht und als Substrat Elastin mit einem chromogenen synthetischen Substrat markiert. Das System wurde mit den Wirkstoffen über 30 min bei Raumtemperatur inkubiert und anschließend nach Zentrifugation die optische Dichte des Farbstoffes bei 410 nm bestimmt. Die Einsatzmenge der Extrakte betrug 0,3 Gew.-%. Die Ergebnisse sind in Tabelle 10 zusammengefaßt. Die Angabe erfolgte relativ zu einer Kontrolle als Standard (= 0 %), als Standard diente α1-Antitrypsin.

**Tabelle 10 Elastaseinhibierung**

| **Extrakt nach Beispiel 2** | **Inhibierung [%]** |
|---|---|
| Batch B | 34 |
| Batch C | 54 |

Die Ergebnisse verdeutlichen, dass Extrakte aus Cassia alata in der Lage sind, die Elastase und speziell die Pankreas-Elastase zu inhibieren. Es kann u. a. auf eine Inibierung der Freisetzung der Elastase zurückgeführt werden.

### 9. Beispiel: Inhibierung der Glycation von Collagen.

Zum Nachweis, dass die Extrakte aus Cassia alata die nicht-enzymatische Glycation von Makromolekülen inhibieren, wurde Kollagen des Typs I mit Glucose und den Extrakten über einen Zeitraum von 21 d bei 45 °C behandelt. Anschließend wurden die Suspensionen zentrifugiert und der Gehalt an Schiffschen Basen in der überstehenden Flüssigkeit durch Fluoreszenzmessung bei 430 nm bestimmt. Die Ergebnisse sind in Tabelle 11 zusammengefaßt. Die Angaben beziehen sich wieder auf die Kontrolle als Standards (ohne Extrakt und ohne Glucose).

**Tabelle 11 Inhibierung der Glycation von Collagen (B1 und B2 sind Extrakte von Rohmaterial aus Indien)**

| | **Extrakt nach Beispiel 1** | | **Extrakt nach Beispiel 2** | |
|---|---|---|---|---|
| | **Batch A** | **Batch C** | **Batch B1** | **Batch B2** |
| Kontrolle ohne Glucose | 46 | 54 | 54 | 54 |
| Kontrolle mit Glucose | 100 | 100 | 100 | 100 |
| Glucose + Extrakt0,1 % | 73 | 73 | 66 | 51 |

Die Ergebnisse verdeutlichen, dass Extrakte aus Cassia alata in der Lage sind, die Glycation von Kollagen zu inhibieren und damit den Alterungsprozess der Dermis durch Glycation der Collagenfasem zu inhibieren.

Die Wirkungen und positiven Aktivitäten der Extrakte aus Cassia alata enthalten folglich eine sehr starke
- anregende (stimulierende), revitalisierende und regenerierende Aktivität auf den Stoffwechsel und damit eine anti-ageing Aktivität
- eine Apoptose inhibierende Aktivität und damit eine anti-ageing Aktivität und
- Zellschutzwirkung gegen Entzündungen besonders bei Entzündungen die durch UVB-Strahlung induziert werden
- Radikal fangende Eigenschaften
- Zellschutzwirkung gegen oxidativen Stress, insbesondere durch UVA-Strahlung ausgelöster Stress
- Proteolyse- und Glycation inhibierende Aktivität

### 10. Beispielrezepturen kosmetischer Mittel mit Cassia alata Extrakten

Die gemäß Beispiel 1 und 2 erhaltenen Cassia alata Extrakte wurden in den folgenden erfindungsgemäßen Rezepturen K1 bis K21 sowie 1 bis 23 eingesetzt. Die so hergestellten kosmetischen Mittel zeigten gegenüber den Vergleichsrezepturen V1, V2 und V3 sehr gute hautpflegende Eigenschaften bei gleichzeitig guter Hautverträglichkeit. Darüber hinaus sind die erfindungsgemäßen Mittel stabil gegen oxidative Zersetzung.

**Tabelle 12: Softcreme Rezepturen K1 bis K7**

| (Alle Angaben in Gew.-% bezogen auf das kosmetische Mittel) | | | | | | | | |
|---|---|---|---|---|---|---|---|---|
| INCl Bezeichnung | K1 | K2 | K3 | K4 | K5 | K6 | K7 | V1 |
| Glyceryl Stearate (and) Ceteareth-12/20 (and) Cetearyl Alcohol (and) Cetyl Palmitate | 8,0 | 8,0 | 8,0 | 8,0 | 8,0 | 8,0 | 8,0 | 8,0 |
| Cetearyl Alcohol | 2,0 | 2,0 | 2,0 | 2,0 | 2,0 | 2,0 | 2,0 | 2,0 |
| Dicaprylyl Ether | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetearyl Isononanoate | 3,0 | 3,0 | 3,0 | 3,0 | 3,0 | 3,0 | 3,0 | 3,0 |
| Glycerin (86 Gew.-%ig) | 3,0 | 3,0 | 3,0 | 3,0 | 3,0 | 3,0 | 3,0 | 3,0 |
| Extrakt nach Beispiel 1 oder 2 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

**Tabelle 13: Nachtcremerezepturen K8 bis K14**

| (Alle Angaben in Gew.-% bezogen auf das kosmetische Mittel) | | | | | | | | |
|---|---|---|---|---|---|---|---|---|
| INCI Bezeichnung | K8 | K9 | K10 | K11 | K12 | K13 | K14 | V2 |
| Polyglyceryl-2 Dipolyhydroxystearate | 4,0 | 4,0 | 4,0 | 4,0 | 4,0 | 4,0 | 4,0 | 5,0 |
| Polyglyceryl-3 Diisostearate | 2,0 | 2,0 | 2,0 | 2,0 | 2,0 | 2,0 | 2,0 | 2,0 |
| Cera Alba | 2,0 | 2,0 | 2,0 | 2,0 | 2,0 | 2,0 | 2,0 | 2,0 |
| Zinc Stearate | 2,0 | 2,0 | 2,0 | 2,0 | 2,0 | 2,0 | 2,0 | 2,0 |
| Cocoglycerides | 3,0 | 3,0 | 3,0 | 3,0 | 3,0 | 3,0 | 3,0 | 3,0 |
| Cetaeryllsononanoate | 8,0 | 8,0 | 8,0 | 8,0 | 8,0 | 8,0 | 8,0 | 8,0 |
| Ether | 5,0 | 5,0 | 5,0 | 5,0 | 5,0 | 5,0 | 5,0 | 5,0 |
| Dicaprylyl Magnesiumsulfate | 1,0 | 1,0 | 1,0 | 1,0 | 1,0 | 1,0 | 1,0 | 1,0 |
| Glycerin (86 Gew.-%ig) | 5,0 | 5,0 | 5,0 | 5,0 | 5,0 | 5,0 | 5,0 | 5,0 |
| Extrakt nach Beispiel 1 oder 2 | 0,5 | 0,5 | 0,5 | 0,5 | 0,5 | 0,5 | 0,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

**Tabelle 14: W/O Bodylotion Rezepturen K15 bis K21**

| (Alle Angaben in Gew.-% bezogen auf das kosmetische Mittel) | | | | | | | | |
|---|---|---|---|---|---|---|---|---|
| INCI-Bezeichnung | K15 | K16 | K17 | K18 | K19 | K20 | K21 | V3 |
| PEG-7 Hydrogenated Castor Oil | 7,0 | 7,0 | 7,0 | 7,0 | 7,0 | 7,0 | 7,0 | 7,0 |
| Decyl Oleate | 7,0 | 7,0 | 7,0 | 7,0 | 7,0 | 7,0 | 7,0 | 7,0 |
| Cetearyl Isononanoate | 7,0 | 7,0 | 7,0 | 7,0 | 7,0 | 7,0 | 7,0 | 7,0 |
| Glycerin (86 Gew.-%ig) | 5,0 | 5,0 | 5,0 | 5,0 | 5,0 | 5,0 | 5,0 | 5,0 |
| MgSO₄ · 7H₂O | 1,0 | 1,0 | 1,0 | 1,0 | 1,0 | 1,0 | 1,0 | 1,0 |
| Extrakt nach Beispiel 1 oder 2 | 1,5 | 1,5 | 1,5 | 1,5 | 1,5 | 1,5 | 1,5 | - |
| Tocopherol | | 0,5 | | | | | | |
| Allantoin | | | 0,2 | | | | | |
| Bisabolol | | | | 0,5 | | | | |
| Chitosan (Hydagen CMF) | | | | | 10,0 | | | |
| Desoxyribonucleinsäure ¹⁾ | | | | | | 0,5 | | |
| Panthenol | | | | | | | 0,5 | |
| Wasser | | | | Ad 100 | | | | |

| | | | | | | | | |
|---|---|---|---|---|---|---|---|---|
| ¹⁾ Desoxyribonucleinsäure: Molekuargewicht ca. 70000, Reinheit (bestimmt durch spektro-photometrische Messung der Absorption bei 260 nm sowie 280 nm): mindestens 1,7. | | | | | | | | |

**Tabelle 15**

| **Kosmetische Zubereitungen** (Alle Angaben in Gew.-% bezogen auf das kosmetische Mittel, Wasser, Konservierungsmittel addieren sich zu 100 Gew.-%) | | | | |
|---|---|---|---|---|
| **Zusammengetzung (INCI)** | **1 Gew.-%** | **2 Gew.-%** | **3 Gew.- %** | **4 Gew.-%** |
| **Texapon® NSO** Sodium Laureth Sulfate | 38,0 | 38,0 | 25,0 | - |
| **Texapon® SB 3** Disodium Laureth Sulfosuccinate | - | - | 10,0 | - |
| **Plantacare® 818** Coco Glucosides | 7,0 | 7,0 | 6,0 | - |
| **Plantacare® PS 10** Sodium Laureth Sulfate (and) Coco Glucosides | - | - | - | 20,0 |
| **Dehyton® PK 45** Cocamidopropyl Betaine | - | - | 10,0 | |
| **Lamesoft® PO 65** Coco-Glucosid (and) Glyceryl Oleate | 3,0 | | | 4,0 |
| **Lamesoft® LMG** Glyceryl Laurate (and) Potassium Cocoyl Hydrolyzed Collagen | - | 5,0 | - | |
| **Euperlan® PK 3000 AM** Glycol Distearate (and) Laureth-4 (and) Cocamidopropyl Betaine | - | 3,0 | 5,0 | 5,0 |
| **Extrakt aus Cassia alata nach Beispiel 1 oder 2** | 1,0 | 1,0 | 1,0 | 1,0 |
| **Arlypon® F** Laureth-2 | 3,0 | 3,0 | 1,0 | - |
| **Sodium Chloride** | - | 1,5 | - | 1,5 |

| | | | | |
|---|---|---|---|---|
| (1-2) Duschbad, (3) Duschgel, (4) Waschlotion | | | | |

**Tabelle 15**

| **Kosmetische Zubereitungen Duschbad "Two in One"** (Alle Angaben in Gew.-% bezogen auf das kosmetische Mittel, Wasser, Konservierungsmittel addieren sich zu 100 Gew.-%) | | | | |
|---|---|---|---|---|
| **Zusammensetzung (INCI)** | **5** | **6** | **7** | **8** |
| **Texapon® NSO** Sodium Laureth Sulfate | 30,0 | 25,0 | | 25,0 |
| **Plantacare® 818** Coco Glucosides | | | | 8,0 |
| **Plantacare® 2000** Decyl Glucoside | | 8,0 | | |
| **Plantacare® PS 10** Sodium Laureth Sulfate (and) Coco Glucosides | | | 20,0 | |
| **Dehyton® PK 45** Cocamidopropyl Betaine | | 10,0 | 10,0 | |
| **Lamesoft® PO 65** Coco-Glucosid (and) Glyceryl Oleate | 5,0 | | | |
| **Lamesoft® LMG** Glyceryl Laurate (and) Potassium Cocoyl Hydrolyzed Collagen | | 5,0 | 5,0 | |
| **Gluadin® WQ** Laurdimonium Hydroxapropyll Hydrolyzed Wheat Protein | 3,0 | | | |
| **Gluadin® WK** Sodium Cocoyl Hydrolyzed Wheat Protein | | | | |
| **Euperlan® PK 3000 AM** Glycol Distearate (and) Laureth-4 (and) Cocamidopropyl Betaine | 5,0 | 3,0 | 4,0 | - |
| **Panthenol** | 0,5 | - | - | 0,5 |
| **Extrakt nach Beispiel 1 oder 2** | 1,0 | 1,0 | 1,0 | 1,0 |
| **Arlypon® F** Laureth-2 | 2,6 | 1,6 | - | 1,0 |
| **Sodium Chloride** | - | - | - | - |

**Tabelle 15**

| **Kosmetische Zubereitungen Schaumbad** (Alle Angaben in Gew.-% bezogen auf das kosmetische Mittel, Wasser, Konservierungsmittel addieren sich zu 100 Gew.-%) | | | | | |
|---|---|---|---|---|---|
| **Zusammensetzung** Bezeichnung nach INCI | **9** | **10** | **11** | **12** | **13** |
| **Texapon® NSO** Sodium Laureth Sulfate | - | - 30,0 | 30,0 | - | 25,0 |
| **Plantacare® 818** Coco Glucosides | - | 10,0 | - | - | 20,0 |
| **Plantacare® PS 10** Sodium Laureth Sulfate (and) Coco Glucosides | 22,0 | - | 5,0 | 22,0 | - |
| **Dehyton® PK 45** Cocamidopropyl Betaine | 15,0 | 10,0 | 15,0 | 15,0 | 20,0 |
| **Monomuls® 90-018** Glyceryl Oleate | 0,5 | | | | |
| **Lamesoft® PO 65** Coco-Glucosid (and) Glyceryl Oleate | | 3,0 | | 3,0 | |
| **Cetiol® HE** PEG-7 Glyceryl Cocoate | | | 2,0 | | 2,0 |
| **Nutrilan® I-50** Hydrolyzed Collagen | 5,0 | | | | |
| **Gluadin® W 40** Hydrolyzed Wheat Gluten | | 5,0 | | 5,0 | |
| **Gluadin® WK** Sodium Cocoyl Hydrolyzed Wheat Protein | | | | 7,0 | |
| **Euperlan® PK 3000 AM** Glycol Distearate (and) Laureth-4 (and) Cocamidopropyl Betaine | 5,0 | - | - | 5,0 | - |
| **Arlypon® F** Laureth-2 | | | 1,0 | | |
| **Sodium Chloride** | 1,0 | | 1,0 | | 2,0 |
| **Extrakte aus Cassia alata nach Beispiel 1 oder 2** | 1,0 | 1,0 | 1,0 | 1,0 | 1,0 |

**Tabelle 15: Kosmetische Zubereitungen (Alle Angaben in Gew.-% bezogen auf das kosmetische Mittel, Wasser, Konservierungsmittel addieren sich zu 100 Gew.-%)**

| **Zusammensetzung (INCI)** | **14** | **15** | **16** | **17** | **18** | **19** | **20** | **21** | **22** | **23** |
|---|---|---|---|---|---|---|---|---|---|---|
| **Dehymuls® PGPH** Polyglyceryl-2 Dipolyhydroxystearate | 4,0 | 3,0 | - | 5,0 | - | - | - | - | - | - |
| **Lameform® TGI** Polyglyceryl-3 Diisostearate | 2,0 | 1,0 | - | - | - | - | - | - | - | - |
| **Emulgade® PL 68150** Cetearyl Glucoside (and) Cetearyl Alcohol | - | - | - | - | 4,0 | - | - | - | 3,0 | - |
| **Eumulgin®B2** Ceteareth-20 | - | - | - | - | - | - | - | 2,0 | - | - |
| **Tegocare® PS** Polyglyceryl-3 Methylglucose Distearate | - | - | 3,0 | - | - | - | 4,0 | - | - | - |
| **Eumulgin VL 75** Polyglyceryl-2 Dipolyhydroxystearate (and) Lauryl Glucoside (and) Glycerin | - | - | - | - | - | 3,5 | - | - | 2,5 | - |
| **Bees Wax** | 3,0 | 2,0 | 5,0 | 2,0 | - | - | - | - | - | - |
| **Cutina® GMS** Glyceryl Stearate | - | - | - | - | - | 2,0 | 4,0 | - | - | 4,0 |
| **Lanette® 0** Cetearyl Alcohol | - | - | 2,0 | - | 2,0 | 4,0 | 2,0 | 4,0 | 4,0 | 1,0 |
| **Antaron® V216** PVP / Hexadecene Copolymer | - | - | - | - | - | 3,0 | - | - | - | 2,0 |
| **Myritol® 818** Cocoglycerides | 5,0 | - | 10,0 | - | 8,0 | 6,0 | 6,0 | - | 5,0 | 5,0 |
| **Finsolv® TN** C12/15 Alkyl Benzoate | - | 6,0 | - | 2,0 | - | - | 3,0 | - | - | 2,0 |
| **Cetiol® J 600** Oleyl Erucate | 7,0 | 4,0 | 3,0 | 5,0 | 4,0 | 3,0 | 3,0 | - | 5,0 | 4,0 |
| **Cetiol® OE** Dicaprylyl Ether | 3,0 | - | 6,0 | 8,0 | 6,0 | 5,0 | 4,0 | 3,0 | 4,0 | 6,0 |
| **MineralOil** | - | 4,0 | - | 4,0 | - | 2,0 | - | 1,0 | - | - |
| **Cetiol® PGL** Hexadecanol (and) Hexyldecyl Laurate | - | 7,0 | 3,0 | 7,0 | 4,0 | - | - | - | 1,0 | - |
| **Panthenol / Bisabolol** | 1,2 | 1,2 | 1,2 | 1,2 | 1,2 | 1,2 | 1,2 | 1,2 | 1,2 | 1,2 |
| **Extrakt aus Cassia alata (Beispiel 1 oder 2)** | 1,0 | 1,0 | 1,0 | 1,0 | 1,0 | 1,0 | 1,0 | 1,0 | 1,0 | 1,0 |
| **Copherol® F 1300** Tocopherol / Tocopheyl Acetate | 0,5 | 1,0 | 1,0 | 2,0 | 1,0 | 1,0 | 1,0 | 2,0 | 0,5 | 2,0 |
| **Neo Heliopan® Hydro** Sodium Phenylbenzimidazole Sulfonate | 3,0 | - | - | 3,0 | - | - | 2,0 | - | 2,0 | - |
| **Neo Heliopan® 303** Octocrylene | - | 5,0 | - | - | - | 4,0 | 5,0 | - | - | 10,0 |
| **Neo Heliopan® BB** Benzophenone-3 | 1,5 | - | - | 2,0 | 1,5 | - | - | - | 2,0 | - |
| **Neo Heliopan® E 1000** Isoamyl p-Methoxydnnamate | 5,0 | - | 4,0 | - | 2,0 | 2,0 | 4,0 | 10,0 | - | - |
| **Neo Heliopan® AV** Octyl Methoxycinnamate | 4,0 | - | 4,0 | 3,0 | 2,0 | 3,0 | 4,0 | - | 10,0 | 2,0 |
| **Uvinul® T150** Octyl Triazone | 2,0 | 4,0 | 3,0 | 1,0 | 1,0 | 1,0 | 4,0 | 3,0 | 3,0 | 3,0 |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5,0 |

| | | | | | | | | | | |
|---|---|---|---|---|---|---|---|---|---|---|
| (14) WIO-Sonnenschutzcreme, (15-17) W/O-Sonnenschutzlo6on, (18, 21, 23) O/W-Sonnenschutzlotion (19, 20, 22) O/W-Sonnenschutzcreme Alle in der Tabelle 12 bis 15 aufgeführten und verwendeten Substanzen mit registriertem Warenzeichen ® sind Marken und Produkte der COGNIS Gruppe. | | | | | | | | | | |

## Patentansprüche

1. Kosmetische Verwendung von Extrakten der Blätter der Pflanze Cassia alata zur vorbeugenden Behandlung von Alterserscheinungen der Haut, wobei die Extrakte in kosmetischen Pflegemitteln verwendet werden.

2. Verwendung nach Anspruch 1, **dadurch gekennzeichnet, dass** die durch UV-Strahlung induzierten Alterserscheinungen der Haut behandelt werden.

## Claims

1. The cosmetic use of extracts of the leaves of the plant Cassia alata for the preventive treatment of signs of skin ageing, wherein the extracts are used in cosmetic care preparations.

2. The use claimed in claim 1, **characterized in that** UV-induced signs of skin ageing are treated.

## Revendications

1. Utilisation cosmétique d'extraits de feuilles de la plante Cassia alata pour le traitement préventif de phénomènes de vieillissement de la peau, les extraits étant utilisés dans des agents de soin cosmétiques.

2. Utilisation selon la revendication 1, **caractérisée en ce qu'**on traite les phénomènes de vieillissement de la peau induite par le rayonnement UV.
